# EUROPÄISCHE PATENTSCHRIFT

(11) **EP 2 199 834 B1**
(45) Veröffentlichungstag und Bekanntmachung des Hinweises auf die Patenterteilung: **25.07.2012**
(21) Anmeldenummer: 09178944.6
(22) Anmeldetag: 11.12.2009
(51) Int. Cl.: G02B 1/04, A61F 2/16, C08F 220/26

(54) **Ophthalmologische Zusammensetzung und ophthalmologische Linse**
Ophthalmological composition and ophthalmologic lens
Composition ophtalmologique et lentille ophtalmique

(30) Priorität: 18.12.2008 DE 102008063742
(43) Veröffentlichungstag der Anmeldung: 23.06.2010
(73) Patentinhaber: Carl Zeiss Meditec AG, 07745 Jena (DE)
(72) Erfinder: Nachbaur, Jürgen, 13509 Berlin (DE)
(74) Vertreter: Hofstetter, Alfons J.

(56) Entgegenhaltungen:
- WO-A1-2007/147599
- WO-A2-2009/074520
- US-A1- 2002 019 492
- US-A1- 2005 055 091
- US-A1- 2006 252 844

## Beschreibung

### Technisches Gebiet

Die Erfindung betrifft eine ophthalmologische Zusammensetzung sowie eine ophthalmologische Linse.

### Stand der Technik

Es ist bekannt, dass violettes Licht, d. h. Licht im Wellenlängenbereich zwischen etwa 400-430 nm, eine ausgeprägte Rolle bei der altersbedingten Makuladegeneration spielt. Unter Makuladegeneration wird dabei eine Gruppe von Erkrankungen des menschlichen und tierischen Auges verstanden, welche mit einem allmählichen Funktionsverlust der im Auge befindlichen Gewebe verbunden ist. Ausgangspunkt der Krankheit ist der Umstand, dass die Einstrahlung von violettem Licht zu einer Akkumulation von molekularem Abfall (Lipofuszin) führt. Dieses Lipofuszin führt in weiterer Konsequenz zur Bildung sogenannter Drusen, die ihrerseits die Makuladegeneration induziert, die bis zur vollständigen Blindheit führen kann. Aus diesem Grund wird violettes Licht als phototoxisch angesehen.

Des Weiteren ist es bekannt, dass blaues Licht, d. h. Licht im Wellenlängenbereich zwischen etwa 450-500nm, für das Sehen bei verminderten Lichtverhältnissen, insbesondere für das Nacht- und Dämmerungssehen, von großer Bedeutung ist. Hierbei spielt das in der Retina des Auges vorkommende Sehpigment Rhodopsin eine wichtige Rolle. Rhodopsin, welches zur Gruppe der G-Protein gekoppelten Rezeptoren zählt, ist dabei in den Photorezeptoren (Stäbchen) der Netzhaut lokalisiert. Ein Einfall von Licht führt zu einer Isomerisierung des vom Rhodopsin gebundenen Retinals (11-cis-Retinal -> all-trans-Retinal).

Ophthalmologische Linsen sind aus dem Stand der Technik in verschiedenen Ausgestaltungen bekannt. Als Intraokularlinsen (IOLs) werden sie in der Regel nach Entfernung der natürlichen Linse (Aphakie) oder bei Fehlsichtigkeiten implantiert. IOLs, welche üblicherweise einen optischen und einen nicht-optischen (haptischen) Teil umfassen, werden unter anderem anhand der zu ihrer Herstellung verwendeten ophthalmologischen Zusammensetzung unterschieden. Man unterscheidet insbesondere zwischen IOLs, die aus einem Acrylat- bzw. Methacrylat-Material gefertigt sind, und IOLs, die aus Silikon-Material gefertigt sind. Weiterhin können IOLs einteilig oder mehrteilig ausgebildet sein. Bei einteiligen Intraokularlinsen bestehen die optischen und die nicht-optischen Teile aus einem einzigen Werkstoff. Bei mehrteiligen IOLs können die optischen und die nicht-optischen Teile aus verschiedenen Werkstoffen bestehen. Die nicht-optischen Teile werden auch als haptische Teile bezeichnet und dienen der Befestigung. Alternativ können die ophthalmologischen Linsen auch als Kontaktlinsen ausgebildet sein, die dementsprechend nur für einen zeitweisen Verbleib auf dem Auge ausgelegt sind.

Um eine Beschädigung der verschiedenen Gewebe im Auge zu verhindern, umfassen die ophthalmologischen Linsen bzw. die zu ihrer Herstellung verwendeten ophthalmologischen Zusammensetzungen üblicherweise einen Farbstoff als Absorber, der violettes Licht möglichst weitgehend absorbieren soll. Der Farbstoff ist im Fall einer Ausgestaltung der Linse als Intraokularlinse (IOL) insbesondere in deren optischem Bereich vorgesehen.

Aus der WO 2009/074520 A2 ist eine ophthalmologische Zusammensetzung bekannt, welche ein Copolymer mit einer Wasseraufnahmefähigkeit zwischen 1 und 59 Gewichtsprozent aufweist.

Die WO 2007/147599 A1 offenbart eine ophthalmologische Zusammensetzung, welche einen UV- und einen Violett-Absorber aufweist.

Auf dem Markt befindliche Farbstoffe für Intraokularlinsen (IOLs) absorbieren jedoch insbesondere im violetten Lichtbereich nur teilweise. Größenordnungsmäßig treten 25% bis 35% des phototoxischen Lichts durch das mit herkömmlichen Farbstoffen versehene Linsenmaterial hindurch. Zudem sind die bekannten Farbstoffe jeweils nur für einen Linsenmaterialtyp geeignet, so dass sie entweder für ophthalmologische Zusammensetzungen auf Acrylat /Methacrylatbasis oder für ophthalmologische Zusammensetzungen auf Silikon-Basis verwendbar sind.

Umgekehrt sollte der Farbstoff bzw. die mit diesem versehene ophthalmologische Linse blaues Licht so wenig wie möglich absorbieren, um eine maximale Lichteinstrahlung und damit ein optimales Dämmerungssehen sicherstellen zu können.

Auf dem Markt befindliche Farbstoffe für IOLs weisen in diesem Wellenlängenbereich (z.B. bei 475 nm) jedoch nur eine Transmission von etwa 70% bis 75% auf.

### Darstellung der Erfindung

Die Aufgabe der vorliegenden Erfindung ist es, eine ophthalmologische Linse mit einem Farbstoff bereitzustellen, welche flexibler verwendbar ist und violettes Licht im Wellenlängenbereich bis etwa 430 nm zumindest überwiegend absorbiert, für blaues Licht im Wellenlängenbereich ab etwa 450-460 nm jedoch zumindest überwiegend durchlässig ist.

Diese Aufgabe wird erfindungsgemäß durch eine ophthalmologische Zusammensetzung gemäß Patentanspruch 1, durch eine ophthalmologische Zusammensetzung gemäß Patentanspruch 2 sowie durch eine ophthalmologische Linse gemäß Patentanspruch 6 gelöst. Vorteilhafte Ausgestaltungen mit zweckmäßigen Weiterbildungen sind in den jeweiligen Unteransprüchen angegeben.

Die Erfindung betrifft eine ophthalmologische Zusammensetzung, welche flexibler verwendbar ist und violettes Licht im Wellenlängenbereich bis etwa 430 nm zumindest überwiegend absorbiert, für blaues Licht im Wellenlängenbereich ab etwa 450-460 nm jedoch zumindest überwiegend durchlässig ist, indem sie einen Farbstoff der allgemeinen Formel I 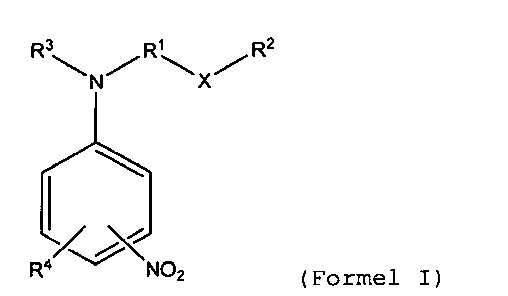 umfasst, wobei der Farbstoff die Struktur 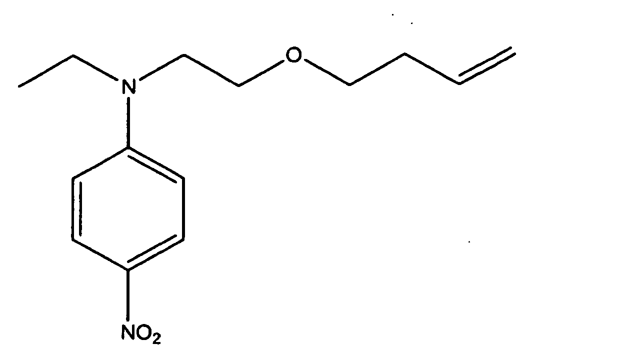 mit:
R¹ = C₂H₄, R² = C₄H₇, R³ = C₂H₅, R⁴ = H und X = O besitzt.

Der Farbstoff eignet sich hierdurch besonders gut für ophthalmologische Zusammensetzungen auf Silikon-Basis und kann beispielsweise zusammen mit Siloxanen, die reaktive Gruppen als endständiges Kettenglied besitzen, polymerisiert werden. Geeignete Siloxane umfassen insbesondere H-Siloxane.

Alternativ ist vorgesehen, dass der Farbstoff der allgemeinen Formel I die Struktur 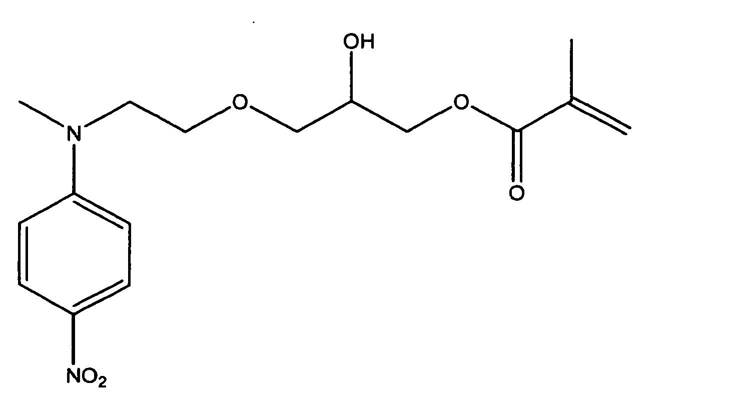 mit:
R¹ = C₅H₁₀O₂, R² = C₄H₅O, R³ = CH₃, R⁴ = H und X = O besitzt. Der Farbstoff eignet sich hierdurch besonders gut für ophthalmologische Zusammensetzungen auf Acrylat- und/oder Methacrylat-Basis und kann beispielsweise zusammen mit hydrophoben und/oder hydrophilen Acrylat- /Methacrylat-Monomeren polymerisiert werden. Grundsätzlich kann anstelle der gezeigten Methacrylat-Gruppe eine Acrylat-Gruppe vorgesehen sein.

Insbesondere ist als eine ophthalmologische Zusammensetzung eine chemische Zusammensetzung oder Zubereitung für die Ophthalmologie zu verstehen.

Dabei sind grundsätzlich alle Enantiomere, Diastereomere und racemischen Gemische der Farbstoffe als mitoffenbart anzusehen. Bei den gelben Farbstoffen handelt es sich mit anderen Worten um polymerisierbare Monomere mit einer Nitroanilin-Gruppe als wesentlichem Strukturelement, welches besonders flexibel für unterschiedliche Polymermaterialien verwendbar ist. Die Farbstoffe absorbieren einerseits violettes Licht im Wellenlängenbereich bis etwa 430 nm zumindest überwiegend, sind aber andererseits zumindest überwiegend durchlässig für blaues Licht im Wellenlängenbereich ab etwa 450-460 nm. Auf diese Weise wird insbesondere bei Verwendung der Farbstoffe für ophthalmologische Zusammensetzungen bzw. für ophthalmologische Linsen ein vorteilhafter Makula-Schutz des Auges eines Patienten bei gleichzeitig gutem Dämmerungssehvermögen gewährleistet.

Unter einer Basis ist im Kontext der vorliegenden Erfindung dabei grundsätzlich ein Gewichtsanteil von wenigstens 51% des gesamten Polymers oder einer gesamten Zusammensetzung zu verstehen. Vorzugsweise ist der Anteil der Basis größer 90%, insbesondere größer 95%, insbesondere größer 98%.

Eine ophthalmologische Zusammensetzung gemäß einem weiteren Aspekt der Erfindung, welche flexibler verwendbar ist und violettes Licht im Wellenlängenbereich bis etwa 430 nm zumindest überwiegend absorbiert, für blaues Licht im Wellenlängenbereich ab etwa 450-460 nm jedoch zumindest überwiegend durchlässig ist, umfasst einen Farbstoff der allgemeinen Formel II 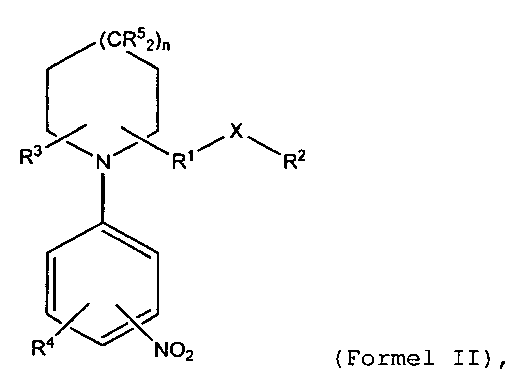 wobei der Farbstoff die Struktur 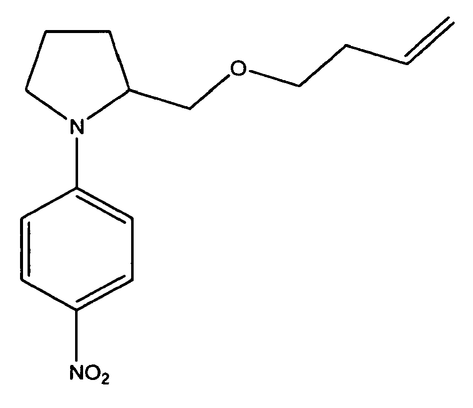 mit:
R¹ = CH₂, R² = C₄H₇, R³ und R⁴ = H, n = 0 und X = O besitzt. Der Farbstoff eignet sich hierdurch besonders gut für ophthalmologische Zusammensetzungen auf Silikon-Basis und kann beispielsweise zusammen mit Siloxanen, die reaktive Gruppen als endständiges Kettenglied besitzen, polymerisiert werden. Geeignete Siloxane umfassen insbesondere H-Siloxane.

Alternativ hat es sich als vorteilhaft gezeigt, wenn der Farbstoff die Struktur 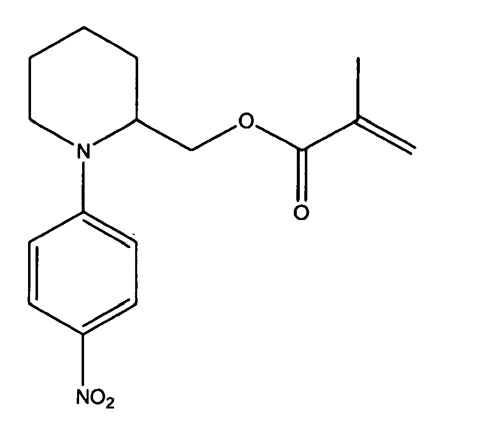 mit:
R¹ = CH₂, R² = C₄H₅O, R³ und R⁴= H, n = 1, R⁵ = H und X = O besitzt. Der Farbstoff eignet sich hierdurch besonders gut für ophthalmologische Zusammensetzungen auf Acrylat- und/oder Methacrylat-Basis und kann beispielsweise zusammen mit hydrophoben und/oder hydrophilen Acrylat-/Methacrylat-Monomeren polymerisiert werden. Grundsätzlich kann anstelle der gezeigten Methacrylat-Gruppe eine Acrylat-Gruppe vorgesehen sein.

Dabei sind grundsätzlich alle Enantiomere, Diastereomere und racemischen Gemische der Farbstoffe der allgemeinen Formel II als mitoffenbart anzusehen. Bei den gelben Farbstoffen der allgemeinen Formel II handelt es sich mit anderen Worten um polymerisierbare Monomere mit einer Nitroanilin-Gruppe und einer gesättigten Ringstruktur als wesentliche Strukturelemente, welches besonders flexibel für unterschiedliche Polymermaterialien verwendbar sind. Die Farbstoffe absorbieren einerseits violettes Licht, sind aber andererseits zumindest überwiegend durchlässig für blaues Licht. Auf diese Weise wird insbesondere bei Verwendung der Farbstoffe für ophthalmologische Zusammensetzungen bzw. für ophthalmologische Linsen ein vorteilhafter Makula-Schutz des Auges eines Patienten bei gleichzeitig gutem Dämmerungssehvermögen gewährleistet.

Dabei hat es sich als vorteilhaft gezeigt, wenn der Farbstoff kovalent in ein Polymer eingebunden ist. Dies minimiert oder verunmöglicht eine unerwünschte Freisetzung des Farbstoffs in vielen Anwendungen besonders zuverlässig.

Vorzugsweise umfasst die ophthalmologische Zusammensetzung ein Monomer gemäß der nachfolgenden allgemeinen Formel III 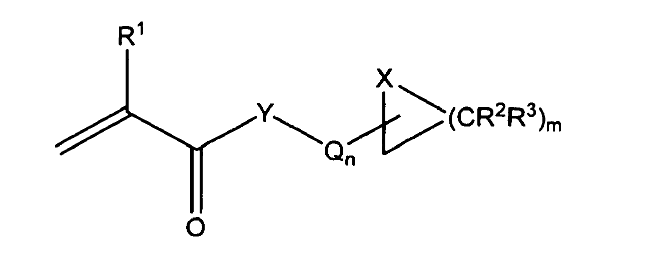 (allgemeine Formel III),
wobei
R¹, R² und R³ jeweils unabhängig voneinander Wasserstoff oder Alkyl- bedeuten;
Y: O oder NR⁴ mit R⁴ ausgewählt aus Wasserstoff oder Alkyl- bedeutet;
X: O, S, SO oder SO₂ bedeutet;
Q: eine Struktureinheit ausgewählt aus CHR⁵ oder (CHR⁵CHR⁶O)ᵢCH₂ bedeutet, wobei R⁵ und R⁶ jeweils unabhängig voneinander Wasserstoff oder Alkyl- bedeuten;
n und i unabhängig voneinander eine ganze Zahl zwischen 1 und 10 und
m eine ganze Zahl zwischen 2 und 6 bedeuten. Mit Hilfe des Monomers der allgemeinen Formel III können ophthalmologische Zusammensetzungen bzw. ophthalmologische Linsen hergestellt werden, die sich besonders gut für die sogenannte Micro Incision Cataract Surgery (MICS) eignen. Dies bedeutet, dass eine Implantation einer entsprechenden ophthalmologischen Linse durch eine Inzision von weniger als 2,0 mm, insbesondere von weniger als 1,7 mm möglich ist.

In einer vorteilhaften Ausgestaltung ist vorgesehen, dass das Monomer der allgemeinen Formel III folgende Struktur aufweist:

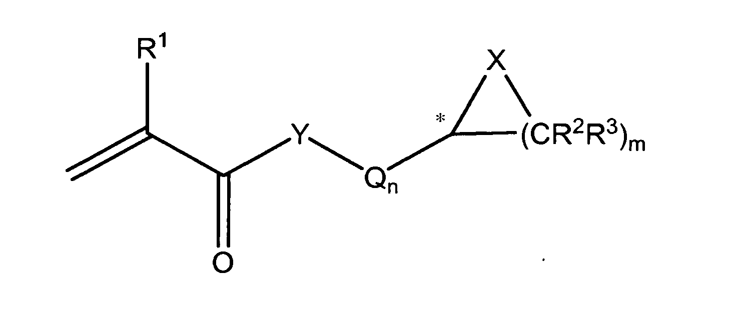

Hierdurch besitzt das Monomer neben den vorstehend erläuterten Vorteilen zusätzlich verbesserte mechanische Eigenschaften, wenn es in eine ophthalmologische Linse eingearbeitet wird.

In einer bevorzugten Ausführungsform sind die Reste R¹, R², R³, R⁴, R⁵ und R⁶ jeweils unabhängig voneinander ausgewählt aus unverzweigten und/oder verzweigten Alkylgruppen mit vorzugsweise 1, 2, 3, 4, 5, 8, 7, 8, 9 und/oder 10 Kohlenstoffatomen. Weiter bevorzugt sind die Reste R¹, R², R³, R⁴, R⁵ und R⁶ unabhängig voneinander eine Methylgruppe, Ethylgruppe, n-Propylgruppe, iso-Propylgruppe, n-Butylgruppe, iso-Butylgruppe und/oder tert-Butylgruppe.

In einer weiter bevorzugten Ausführungsform ist die Struktureinheit Q eine Methylengruppe und in einer weiter bevorzugten Ausführungsform ist die Struktureinheit Q eine -CH(CH₃)CH₂OCH₂- Gruppe.

In einer weiter bevorzugten Ausführungsform sind n und i unabhängig voneinander 1, 2, 3, 4, 5, 8, 7, 8, 9 und/oder 10. In einer weiter bevorzugten Ausführungsform ist m 2, 3, 4, 5 oder 6.

In einer weiter bevorzugten Ausführungsform ist der Rest R¹ eine Methylgruppe, wenn die Struktureinheit Y ein O Atom ist. Es ist weiter bevorzugt, dass der Rest R¹ Wasserstoff darstellt, wenn die Gruppe Y NH ist.

Es ist weiter bevorzugt, dass das Monomer der allgemeinen Formel III folgende Struktur 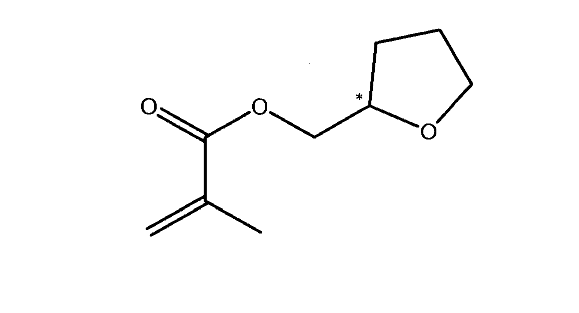 aufweist. Dieses Monomer (Tetrahydrofuran-2-yl)-methylmethacrylat ist dabei auch unter dem Trivialnamen Tetrahydrofurfurylmethacrylat (THFMA) bekannt. Alternativ oder zusätzlich kann hierbei auch das Stellungsisomer (Tetrahydrofuran-3-yl)-methylmethacrylat vorgesehen sein.

Es kann vorgesehen sein, dass die ophthalmologische Zusammensetzung ein Copolymer aufweist, wobei das Copolymer:
a) bezogen auf das Gesamtgewicht des Copolymers 20 bis 95 Gewichtsprozent Struktureinheiten, stammend aus wenigstens einem hydrophilen Monomer, und
b) bezogen auf das Gesamtgewicht des Copolymers 5 bis 80 Gewichtsprozent Struktureinheiten, stammend aus wenigstens einem Monomer gemäß der allgemeinen Formel III 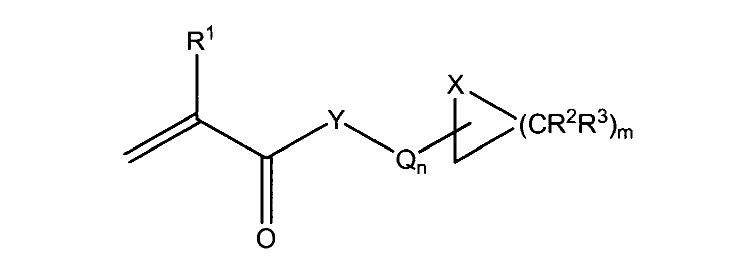 umfasst, wobei
R¹, R² und R³ jeweils unabhängig voneinander Wasserstoff oder Alkyl- bedeuten,
Y: O oder NR⁴ mit R⁴ ausgewählt aus Wasserstoff oder Alkyl- bedeutet,
X: O, S, SO oder SO₂ bedeutet,
Q: eine Struktureinheit ausgewählt aus CHR⁵ oder (CHR⁵CHR⁶O)ᵢCH₂ bedeutet, wobei R⁵ und R⁶ jeweils unabhängig voneinander Wasserstoff oder Alkyl- bedeuten,
n und i unabhängig voneinander eine ganze Zahl zwischen 1 und 10 und
m eine ganze Zahl zwischen 2 und 6 bedeuten,
und wobei das Copolymer bezogen auf das Gesamtgewicht des Copolymeren einen Wassergehalt von 1 bis 59 Gewichtsprozent aufweist. Alle Stereoisomere und racemischen Gemische der Monomeren a) und b) sind dabei grundsätzlich als mitumfasst anzusehen. Insbesondere weist das Copolymer verbesserte Eigenschaften auf, wenn es in eine ophthalmische Linse eingearbeitet bzw. zur Herstellung einer derartigen Linse verwendet wird. Eine solche ophthalmische Linse und insbesondere eine Intraokularlinse lässt sich bei der Implantation besser falten, sodass der chirurgische Eingriff vor Einführung der Intraokularlinse ins Auge einen besonders kleineren Einschnitt verlangt. Die Linse eignet sich daher besonders gut für die sogenannte Micro Incision Cataract Surgery (MICS). Außerdem ist die Verträglichkeit eines solchen Copolymers im Auge sehr gut.

Es ist weiter bevorzugt, dass bezogen auf das Gesamtgewicht des Copolymers 30 bis 79 Gewichtsprozent Struktureinheiten aus dem wenigsten einen hydrophilen Monomer a) und weiter bevorzugt 50 bis 79 Gewichtsprozent Struktureinheiten aus dem wenigstens einen hydrophilen Monomer a) in dem Copolymer stammen.

In einer weiter bevorzugten Ausführungsform stammen bezogen auf das Gesamtgewicht des Copolymers in dem Copolymer 10 bis 79 Gewichtsprozent, insbesondere 21 bis 60 Gewichtsprozent, vorzugsweise 21 bis 50 Gewichtsprozent und weiter bevorzugt 21 bis 35 Gewichtsprozent aus dem wenigstens einen Monomer b) gemäß der allgemeinen Formel III. Insbesondere können auch bezogen auf das Gesamtgewicht des Copolymers in dem Copolymer 10 bis 35 Gewichtsprozent aus dem wenigstens einen Monomer b) gemäß der allgemeinen Formel III stammen.

Vorzugsweise weist das Copolymer einen Wassergehalt von 2 bis 50 Gewichtsprozent, weiter bevorzugt von 5 bis 40 Gewichtsprozent und besonders bevorzugt zwischen 10 und 30 Gewichtsprozent bezogen auf das Gesamtgewicht des Copolymers auf.

Die in im Rahmen der Offenbarung angegebenen Anteile an Struktureinheiten stammend aus Monomeren beziehen sich auf das Gesamtgewicht des Copolymers und diese einzelnen Anteile und der Wassergehalt sind bevorzugt so zu wählen, dass in Summe 100 Gewichtsprozent erhalten werden. Für den Fall, dass weitere Inhaltsstoffe in dem Copolymer enthalten sind, sind diese Gewichtsanteile und der Wassergehalt so zu wählen, dass sich ein Gesamtgewicht des Copolymers inklusive der weiteren Inhaltsstoffe zu 100 Gewichtsprozent ergibt.

Es ist weiter bevorzugt, dass das hydrophile Monomer a) ein Monomer der allgemeinen Formel IV 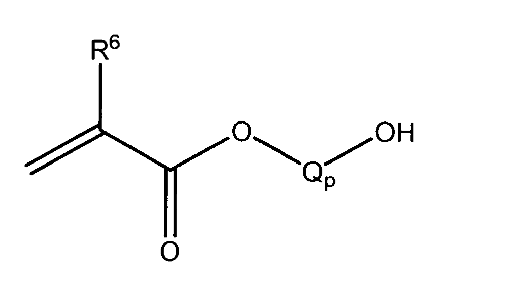 (allgemeine Formel IV)
ist, wobei
Q: eine Struktureinheit ausgewählt aus CHR⁷ oder (CHR⁷CHR⁸0)ₖCH₂ bedeutet, wobei R⁷ und R⁸ jeweils unabhängig voneinander Wasserstoff oder Alkyl- bedeuten, und
p und k unabhängig voneinander eine ganze Zahl zwischen 1 und 10 bedeuten. Auch hierbei sind alle Stereoisomere und racemischen Gemische als mitumfasst anzusehen.

In einer bevorzugten Ausführungsform sind die Reste R⁶, R⁷ und R⁸ jeweils unabhängig voneinander ausgewählt aus unverzweigten und/oder verzweigten Alkylgruppen mit vorzugsweise 1, 2, 3, 4, 5, 8, 7, 8, 9 und/oder 10 Kohlenstoffatomen. Weiter bevorzugt sind die Reste R⁶, R⁷ und R⁸ jeweils unabhängig voneinander eine Methylgruppe, Ethylgruppe, n-Propylgruppe, iso-Propylgruppe, n-Butylgruppe, iso-Butylgruppe und/oder tert-Butylgruppe. Für R⁶ ist unabhängig davon besonders bevorzugt, dass R⁶ Methyl- oder H ist.

Für einige Fälle kann es bevorzugt sein, dass das Copolymer keine Struktureinheiten enthält, die aus wenigstens einem Alkoxyalkyl-Methacrylatmonomer und/oder einem Alkoxyalkyl-Acrylatmonomer stammen.

In einer weiter bevorzugten Ausführungsform sind k und p unabhängig voneinander 1, 2, 3, 4, 5, 8, 7, 8, 9 und/oder 10.

In einer noch weiter bevorzugten Ausführungsform ist das hydrophile Monomer der allgemeinen Formel IV Hydroxyethylmethacrylat (HEMA) und/oder Hydroxypropylmethacrylat (HPMA). Alternativ oder zusätzlich kann Glycerolmonomethacrylat als hydrophiles Monomer vorgesehen sein.

Es ist weiter bevorzugt, dass das Monomer b) der allgemeinen Formel III folgende Struktur 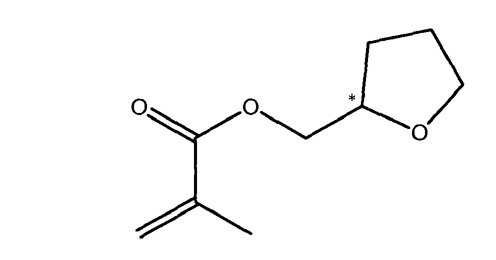 aufweist. Dieses Monomer (Tetrahydrofuran-2-yl)-methylmethacrylat ist dabei auch unter dem Trivialnamen Tetrahydrofurfurylmethacrylat (THFMA) bekannt. Alternativ oder zusätzlich kann hierbei auch das Stellungsisomer (Tetrahydrofuran-3-yl)-methylmethacrylat vorgesehen sein.

In einer bevorzugten Ausführungsform liegen die Monomere der allgemeinen Formel III und/oder IV in enantiomerenreiner Form vor. Alternativ bevorzugt können die Monomere der allgemeinen Formel III und/oder IV als racemisches Gemisch vorliegen.

In einer weiter bevorzugten Ausführungsform umfasst das Copolymer wenigstens einen oder mehrere Vernetzer. Als geeignete Vernetzer können Vinylmonomere oder -oligomere vorgesehen sein, die zwei oder mehr polymerisierbare Gruppen aufweisen. Hierdurch kann das Copolymer gezielt dreidimensional vernetzt und der Vernetzungsgrad optimal in Abhängigkeit des jeweiligen Anwendungszwecks eingestellt werden. Beispielsweise können als Vernetzer Ethylenglycoldimethacrylat (EGDMA), Trimethylolpropantri(meth)acrylat, 1,3-Glycerindi(meth)acrylat und/oder Butandioldi(meth)acrylat vorgesehen sein.

In einer weiter bevorzugten Ausführungsform enthält die ophthalmologische Zusammensetzung einen UV-Absorber. Unter UV-Absorber sind dabei organische oder anorganische Verbindungen zu verstehen, die Strahlung in einem Wellenlängenbereich zwischen 200 nm und 400 nm zumindest überwiegend und vorzugsweise quantitativ absorbieren. Als UV-Absorber ist ein biokompatibles UV-Lichtschutzmittel vorgesehen, wofür Cumarinderivate, die gegebenenfalls über Alkylspacer mit einer bzw. mehreren Acryl- oder Methacrylfunktionen verknüpft sind, verwendet werden.

In einer weiteren vorteilhaften Ausgestaltung der Erfindung ist vorgesehen, dass der UV-Absorber eine Verbindung der allgemeinen Formel V ist, bei der ein Cumarin-Grundkörper über verschiedene Spacer mit einem oder mehreren Acryl- bzw. Methacryl-Resten verbunden ist: 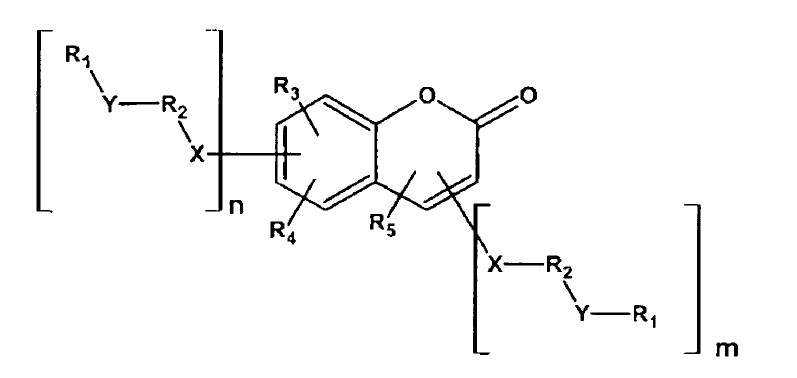 (allgemeine Formel V)
besitzt, wobei
R₁: Acryl- und/oder Methacryl-Reste 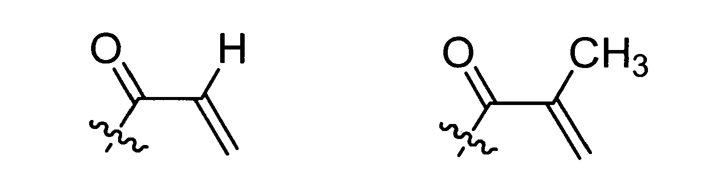 bedeuten,
R₂: organische verzweigte und/oder unverzweigte Alkyl- und/oder Aryl-Substituenten mit bis zu 30 Atomen ausgewählt aus C, H, Si, O, N, P, S, F, Cl, Br bedeuten,
R₃, R₄ und R₅: H und/oder organische verzweigte und/oder unverzweigte Alkyl- und/oder Aryl-Substituenten mit bis zu 30 Atomen ausgewählt aus C, H, Si, O, N, P, S, F, Cl, Br bedeuten,
X, Y: O, S, NH oder NR⁶ bedeuten, wobei R⁶ ein organischer verzweigter und/oder unverzweigter Alkyl- und/oder Aryl-Substituent mit bis zu 30 Atomen ausgewählt aus C, H, Si, O, N, P, S, F, Cl, Br ist, und
n eine ganze Zahl zwischen 0 und 2 sowie m 0 oder 1 ist, wobei die Summe n+m immer größer gleich 1 ist.

Auch hierbei sind alle Stereoisomere und racemischen Gemische als mitumfasst anzusehen. Beispiele für geeignete Strukturen gemäß Formel V sind:

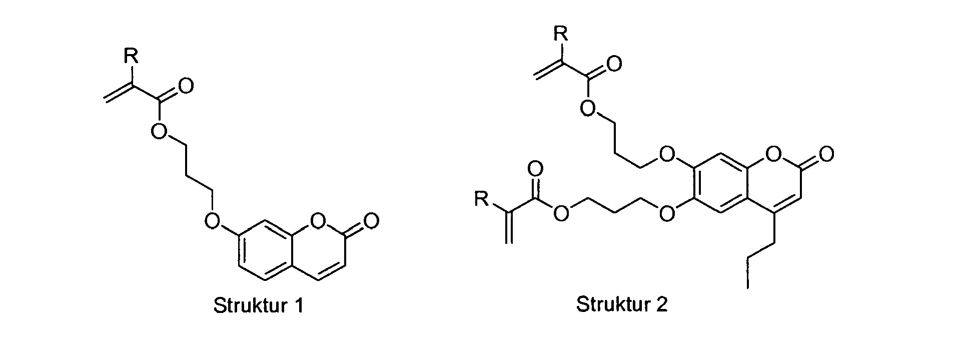

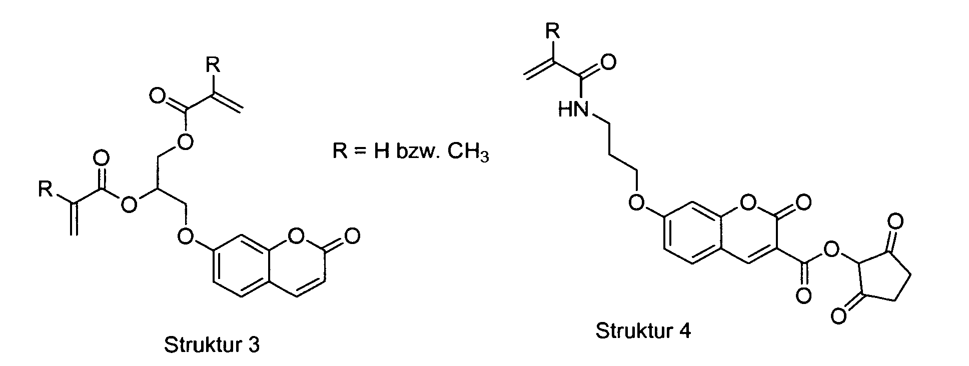

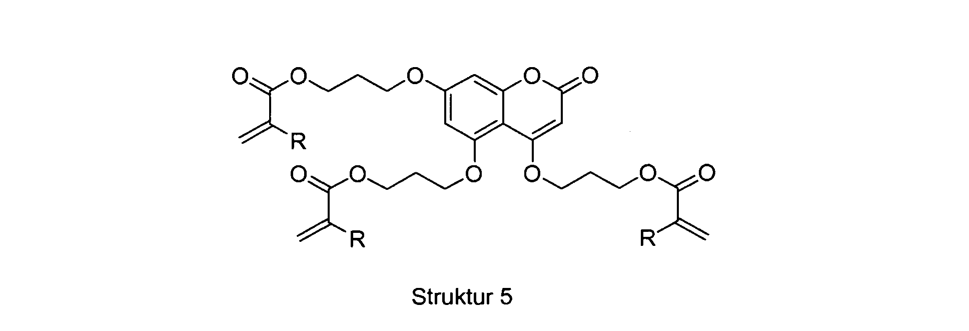

UV-Absorber, deren Grundstruktur auf den Strukturen 2, 3 oder 5 basieren, haben den zusätzlichen Vorteil, dass diese bedingt durch das Vorhandensein mehrerer polymerisierbarer Endgruppen einen quantitativen Einbau in das Linsenmaterial ermöglichen und darüber hinaus vernetzende Eigenschaften besitzen. Bei einer Linsenherstellung kann so im Idealfall auf die Zugabe eines zusätzlichen Vernetzers verzichtet werden.

Ein bevorzugter UV-Absorber ist Cumarin-7-propoxymethacrylat mit n = 1, m = 0, X = O, R₂ = C₃H₆, Y = O, R₁ = Methacrylrest, R₃ = H, R₄ = H, R₅ = H mit der Struktur: 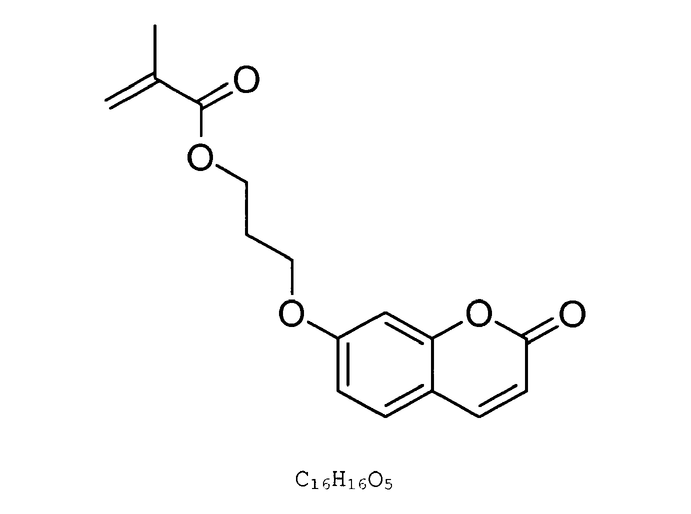 Molekulargewicht 288,30 g/mol

Die Herstellung dieser Verbindung erfolgt in zwei Schritten, wobei das 7-Hydroxycumarin kommerziell erhältlich ist:

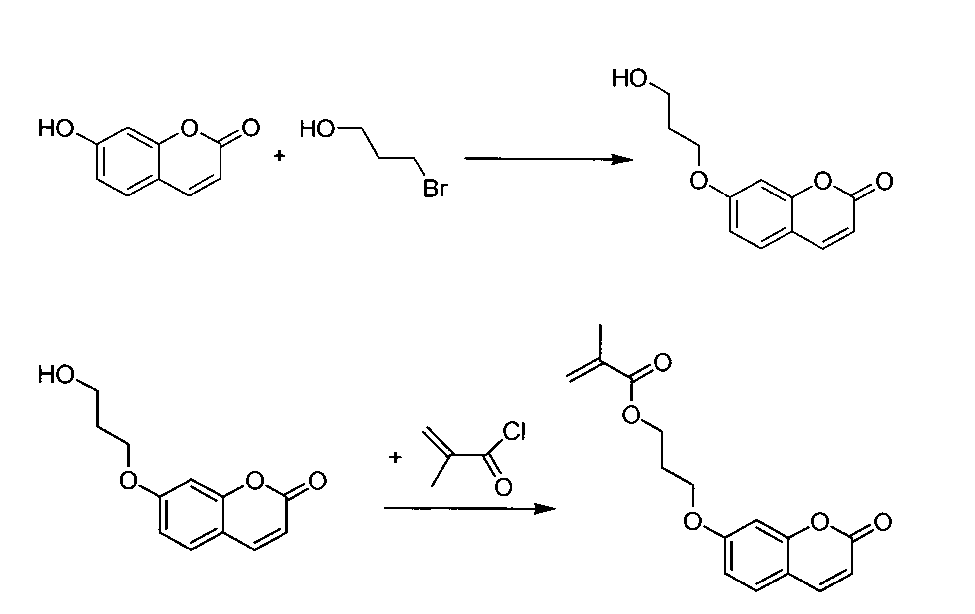

Ein weiteres Beispiel für den UV-Absorber der allgemeinen Formel V ist die Verbindung mit n = 2, m = 0, X = O, R₂ = C₃H₆, Y = O, R₁ = Acryl- und/oder Methacrylrest, R₃ = H, R₄ = H, R₅ = H in der allgemeinen Formel V, wodurch sich die nachfolgende Struktur ergibt:

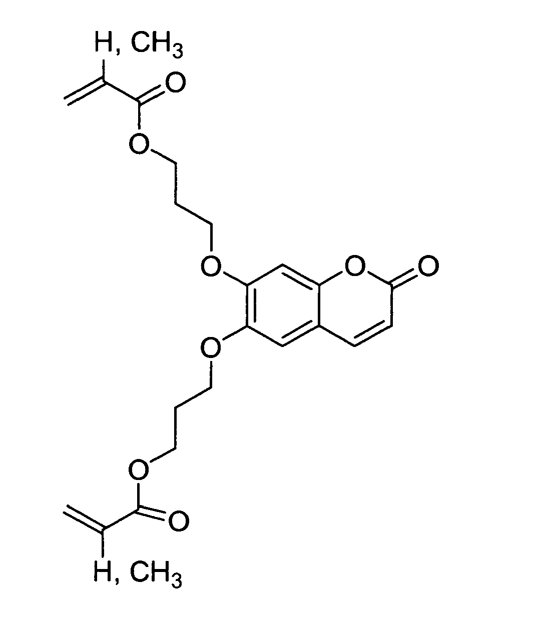

Ein weiteres Ausführungsbeispiel für einen UV-Absorber ist Cumarin-6,7-dipropoxymethacrylat. Auch dieser kann auf einfachem synthetischem Wege analog dem Cumarin-7-propoxymethacrylat in einer 2-stufigen Reaktion dargestellt werden. Das dazu benötigte 6,7-Dihydroxycumarin ist ebenfalls kommerziell verfügbar. Auf diese Weise kann eine Verbindung hergestellt werden, bei der eine zusätzliche Methacrylat-Ankergruppe eingeführt wurde. Das Anbinden dieser zweiten Ankergruppe über einen Alkoxyspacer hat nur einen geringen Einfluss auf die spektralen Eigenschaften des Absorbers, ermöglicht jedoch diesen ebenfalls als Vernetzter bei der Herstellung des Linsenmaterials einzusetzen.

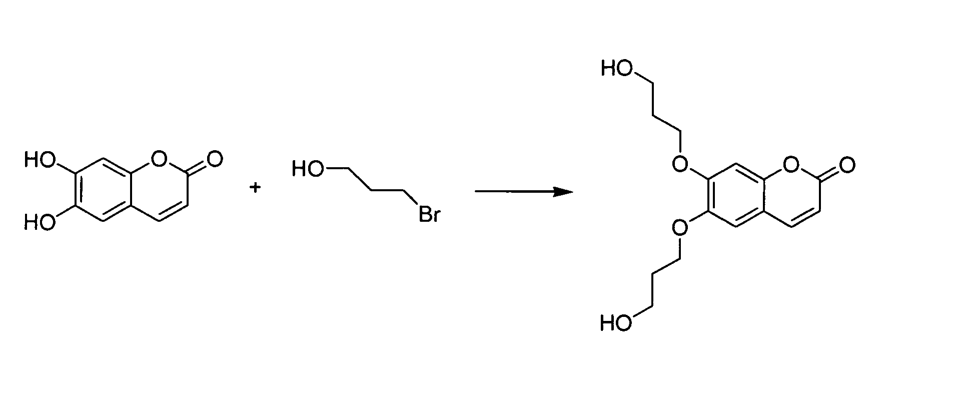

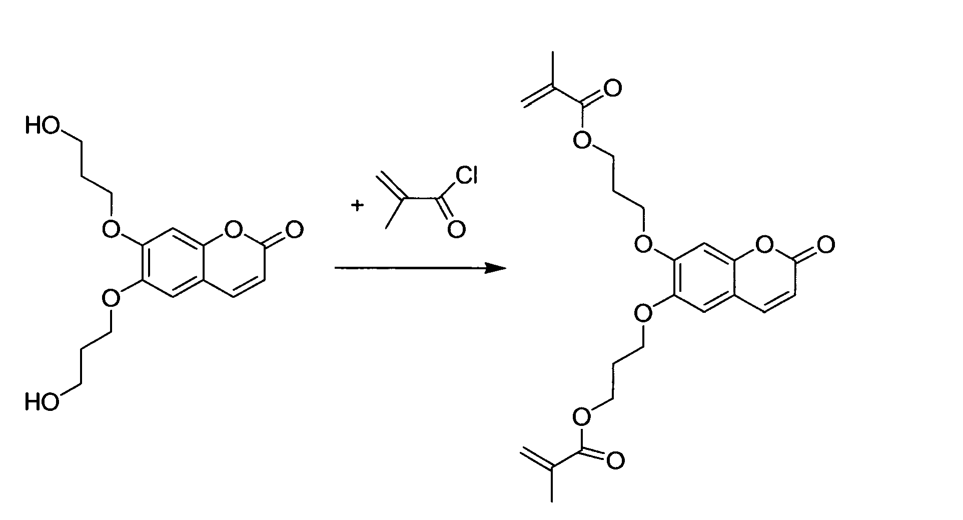

Ein weiteres Beispiel für einen UV-Absorber der allgemeinen Formel V ist eine Struktur mit n = 1, m = 0, X = 0, R₂ = -CH₂-CH(OR₁)CH₂-, Y = O, R₁ = Acryl- bzw. Methacrylrest, R₃ = H, R₄ = H, R₅ = H.

Eine weitere Möglichkeit einen UV-Absorber mit zwei Ankergruppen herzustellen ergibt sich aus der Verwendung eines verzweigten Dihydroxyhalogenids. Setzt man 7-Hydroxycumarin in einem ersten Schritt mit kommerziell erhältlichem 3-Brom-1,2-propandiol um und acryliert bzw. methacryliert das entstandene Alkoxydiol nachfolgend, erhält man einen weiteren bifunktionalen UV-Absorber.

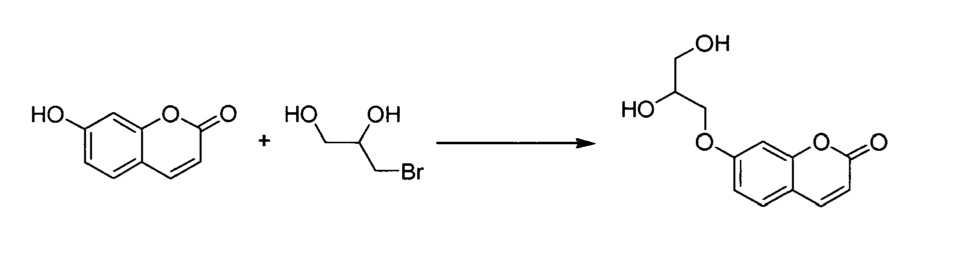

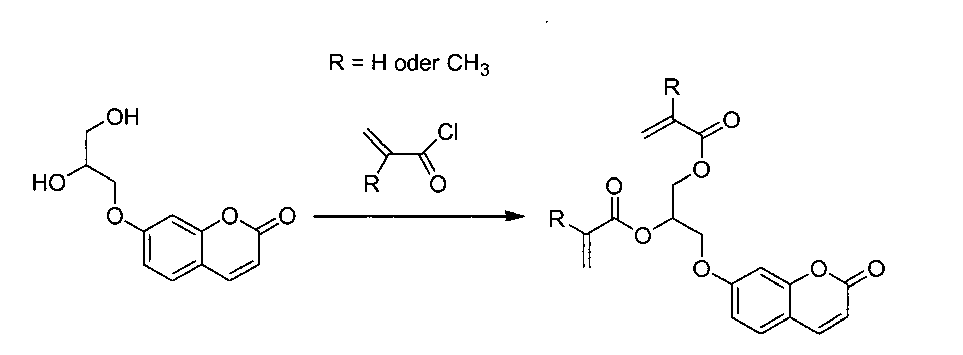

Ein weiteres Beispiel für einen UV-Absorber der allgemeinen Formel V ist eine Struktur mit n = 1, m = 0, X = O, R² = -CH₂-CH(OR1)CH₂-, Y = O, R¹ = Methacrylrest, R³ = H, R⁴ = H, R⁵ = H.

Setzt man 7-Hydroxycumarin nicht mit Acrylsäure bzw. Methacrylsäurechlorid, sondern mit kommerziell erhältlichem Methacrylsäureglycidylester um, so erhält man in einem einzigen Reaktionsschritt einen weiteren UV-Filter, in dem der Cumarin-Grundkörper durch eine aliphatische Kette vom Methacrylatrest getrennt ist. Durch nachfolgende Veresterung mit Methacryloylchlorid kann eine weitere Methacrylatfunktion an der sekundären Alkoholgruppe eingeführt werden.

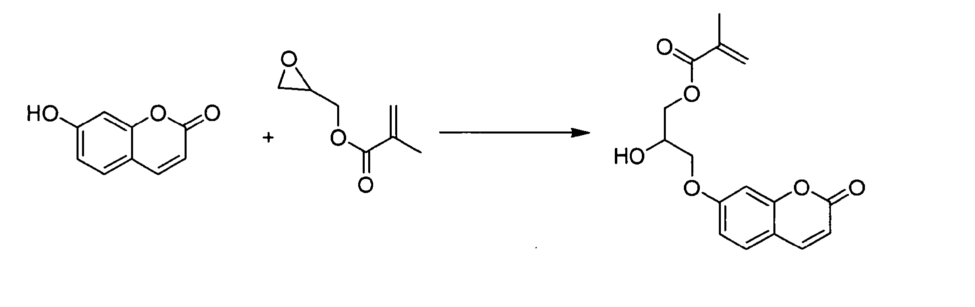

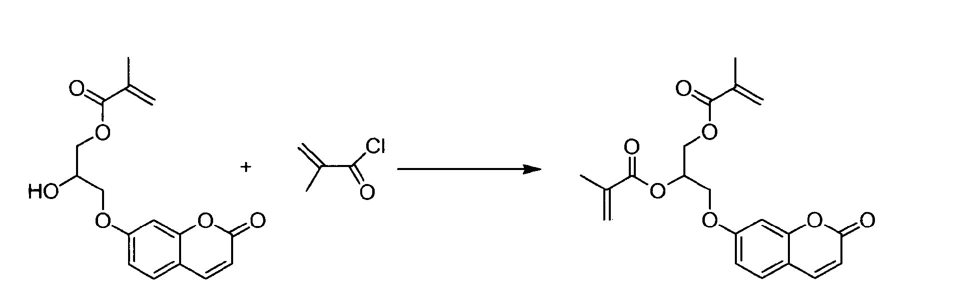

Ein weiteres Beispiel für einen UV-Absorber der allgemeinen Formel V ist eine Struktur mit n = 1, m = 0, X = O, R₂ = C₃H₆, Y = O, R₁ = Acryl- bzw. Methacrylrest, R₃ = H, R₄ = H, R₅ = C₃H₇.

Hier ist R₅ eine Propylgruppe, die einen schwachen induktiven Effekt (+I-Effekt) besitzt. Das Einbringen einer zusätzlichen Propylgruppe in den zuvor beschriebenen bevorzugten UV-Absorber ist synthetisch ohne Probleme zu bewältigen und verändert die spektralen Eigenschaften des Chromophors nur in geringem Maße. Setzt man bei der Synthese nicht 7-Hydroxycumarin sondern das ebenfalls kommerziell erhältliche 7-Hydroxy-4-propylcoumarin ein, erhält man nach der Methacrylierung ein Cumarinderivat, dass sich vom bevorzugten UV-Absorber um nur eine Propylseitenkette unterscheidet.

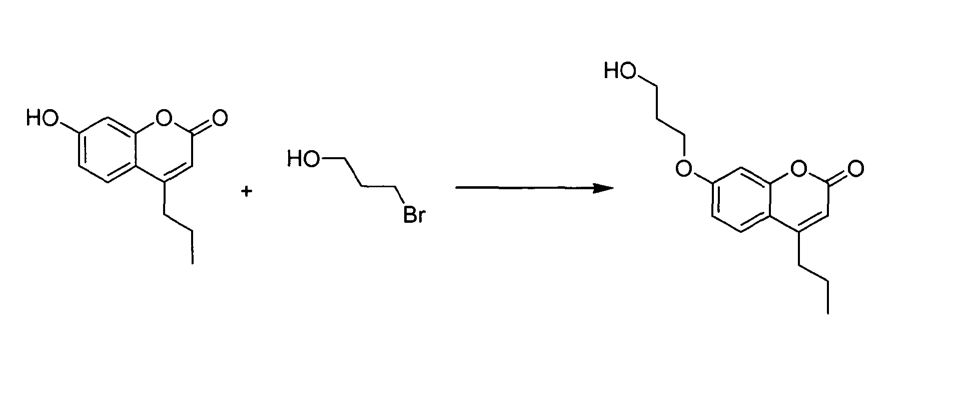

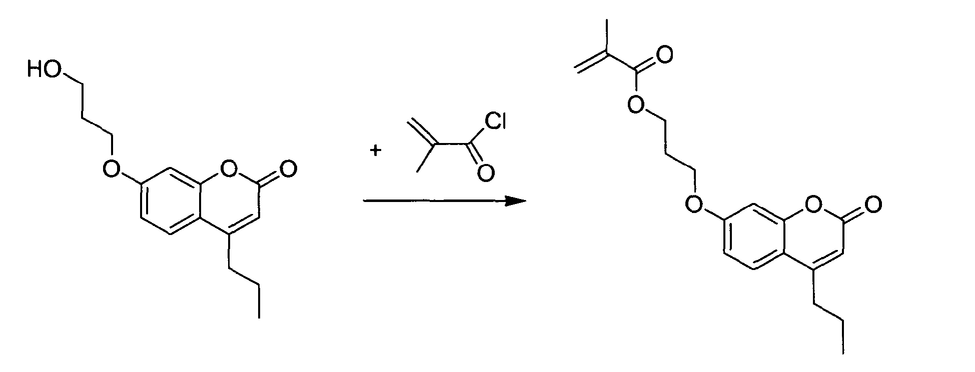

Ein weiteres Beispiel für einen UV-Absorber ist eine Struktur der allgemeinen Formel V mit n = 2, m = 1, X = O, R₂ = C₃H₆, Y = O, R₁ = Acryl- bzw. Methacrylrest, R₃ = H, R₄ = H, R₅ = H.

Auch ein trifunktionaler UV-Absorber lässt sich auf einfachem synthetischem Wege herstellen. Ausgehend vom 4,5,7-Trihydroxycumarin erhält man nach der Alkoxylierung mit 3-Brom-1-propanol und nachfolgender Acrylierung bzw. Methacrylierung einen UV-Absorber der allgemeinen Formel V mit drei Ankergruppen.

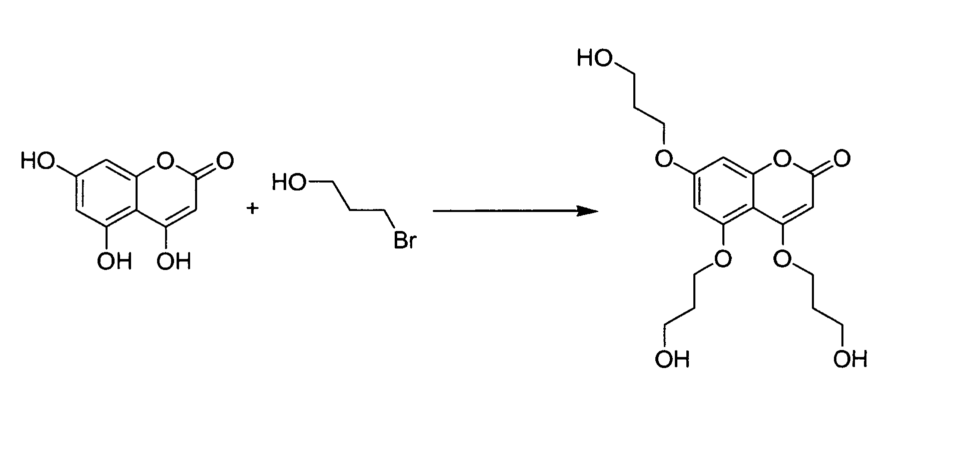

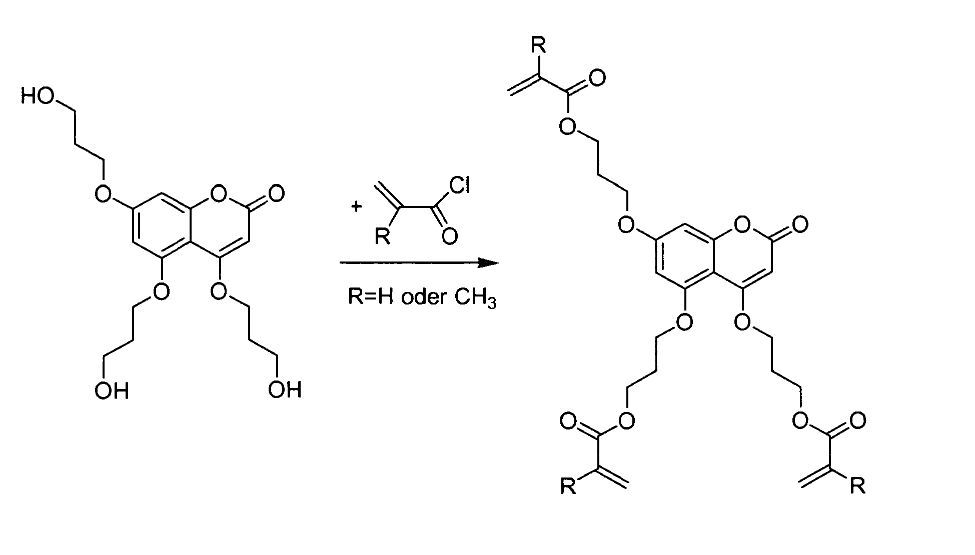

Es kann auch vorgesehen sein, dass der UV-Absorber Methacrylsäure-(2-[3-(2H-benzotriazol-2-yl)4-hydroxyphenyl]ethylester) ist.

Die ophthalmologische Zusammensetzung kann beispielsweise eine folgende Komponentenausgestaltung aufweisen:

| | |
|---|---|
| EOEMA (Ethoxyethylmethacrylat) | 85 - 97 Gew.-% |
| MMA (Methylmethacrylat) | 0 - 15 Gew.-% |
| EEEA (Ethoxyethoxyethylacrylat) | 0 - 5 Gew.-% |
| EGDMA (Ethylenglykoldimethacrylat) | 0 - 0,7 Gew.-% |
| UV-Absorber | 0,1 - 1,0 Gew.-% |
| Violett-Absorber | 0,03 - 0,16 Gew.-% |

Dabei ist der Violett-Absorber ein Farbstoff der Formeln I und/oder II oder eine vorteilhafte Ausführung davon.

In einer weiteren vorteilhaften Ausgestaltung der Erfindung umfasst die ophthalmologische Zusammensetzung 50-90%, vorzugsweise 70-85%, besonders bevorzugt 70-80% HEMA (Hydroxyethylmethacrylat), 10-40%, vorzugsweise 16-40%, vorzugsweise 16-30% MMA (Methylmethacrylat), 0,25-1,5%, vorzugsweise 0,4%-0,6%, insbesondere 0,5% Vernetzer, 0,01-0,3% Radikalstarter, 0,1-3,0%, vorzugsweise 0,70-0,80%, vorzugsweise 0,75% UV-Absorber und 0,02-0,3%, vorzugsweise 0,16-0,30% Farbstoff als Violett-Absorber. Die Prozentangaben stehen dabei grundsätzlich für Gewichtsprozente. Vorzugsweise beträgt der Wassergehalt der Zusammensetzung zwischen 20% und 35%, vorzugsweise zwischen 24% und 30%.

Es kann vorgesehen sein, dass die ophthalmologische Zusammensetzung folgende Komponenten aufweist:

| | |
|---|---|
| HEMA (Hydroxyethylmethacrylat) | 50 - 85 Gew.-% |
| EOEMA (Ethoxyethylmethacrylat) | 30 - 40 Gew.-% |
| THFMA (Tetrahydrofufurylmethacrylat) | 5 - 20 Gew.-% |
| EGDMA (Ethylenglykoldimethacrylat) | 0 - 0,7 Gew.-% |
| UV-Absorber | 0,1 - 1,0 Gew.-% |
| Violett-Absorber | 0,03 - 0,16 Gew.-% |

Dabei ist der Violett-Absorber ein Farbstoff der Formeln I und/oder II oder eine vorteilhafte Ausführung davon.

In einer weiteren Ausführung kann der Anteil des THFMA größer als 20%, insbesondere bis zu 50%, sein, wobei der Anteil der Komponente HEMA dann kleiner als 50% sein kann und/oder der Anteil von EOEMA kleiner als 30% sein kann. EOEMA kann auch nicht vorhanden sein.

Bei allen Ausführungen sind die Gewichtsprozente der Komponenten in der jeweiligen Zusammensetzung in Summe so gewählt, dass sie 100% ergeben, wobei eventuell noch vorhandene Radikalstarter bzw. deren ins Polymer eingebundenen Bestandteile mitumfasst sind.

Ein geeigneter Vernetzer ist beispielsweise EGDMA (Ethylenglykoldimethacrylat). Als Radikalstarter kann - gegebenenfalls in Abhängigkeit der Reaktionstemperaturen - jedoch beispielsweise auch AIBN (Azo-bis-(isobutyronitril)) und/oder V65 vorgesehen sein.

Die ophthalmologische Zusammensetzung besitzt eine hohe Biokompatibilität sowie einen hohen Scheitelbrechwert (Dioptrie). Zudem eignet sich die ophthalmologische Zusammensetzung besonders gut zur Herstellung von ophthalmologischen Linsen für Kleinschnittanwendungen, insbesondere für MICS-Anwendungen. Die ophthalmologische Zusammensetzung besitzt weiterhin vorzugsweise einen Wassergehalt zwischen 20% und 35%, insbesondere zwischen 24% und 30%.

Ein weiterer Aspekt der Erfindung betrifft eine ophthalmologische Linse, welche erfindungsgemäß flexibler verwendbar ist und violettes Licht im Wellenlängenbereich bis etwa 430 nm zumindest überwiegend absorbiert, für blaues Licht im Wellenlängenbereich ab etwa 450-460 nm jedoch zumindest überwiegend durchlässig ist, indem sie eine ophthalmologische Zusammensetzung nach einem der vorhergehenden Ausführungsbeispiele mit einem der vorstehend genannten Farbstoffe der allgemeinen Formel I und/oder der allgemeinen Formel II umfasst.

Die erfindungsgemäße Linse verfügt hierdurch über einen hohen Brechungsindex sowie über eine besonders gute Faltbarkeit bei hoher Biokompatibilität und kann grundsätzlich einteilig oder mehrteilig ausgebildet sein. Dabei kann grundsätzlich vorgesehen sein, dass die Linse ausschließlich aus der ophthalmologischen Zusammensetzung besteht. Weitere Merkmale und deren Vorteile sind der vorhergehenden Beschreibung zu entnehmen und gelten entsprechend für die ophthalmologische Linse, wobei insbesondere die vorteilhaften Ausführungen der ophthalmologischen Zusammensetzung gemäß dem oben genannten Aspekt als vorteilhafte Ausführungen der weiteren ophthalmologischen Zusammensetzung anzusehen sind.

In einer vorteilhaften Ausgestaltung der Erfindung hat es sich als vorteilhaft gezeigt, wenn die Linse als Intraokularlinse ausgebildet ist. Es kann auch eine Ausgestaltung als Kontaktlinse vorgesehen sein.

Ein Farbstoff der allgemeinen Formel I und/oder der allgemeinen Formel II und/oder eine ophthalmologische Zusammensetzung nach einem der vorhergehenden Ausführungsbeispiele kann zur Herstellung einer ophthalmologischen Linse, insbesondere einer Kontakt- oder Intraokularlinse, und/oder eines medizinischen Implantats, insbesondere eines Augenimplantats, verwendet werden. Die sich hieraus ergebenden Merkmale und deren Vorteile sind den vorhergehenden Beschreibungen zu entnehmen.

Weitere Merkmale der Erfindung ergeben sich aus den Ansprüchen und den Ausführungsbeispielen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

### Beispiel 1

Im ersten Beispiel für einen gelben Farbstoff der allgemeinen Formel I wird die folgende Verbindung 1: 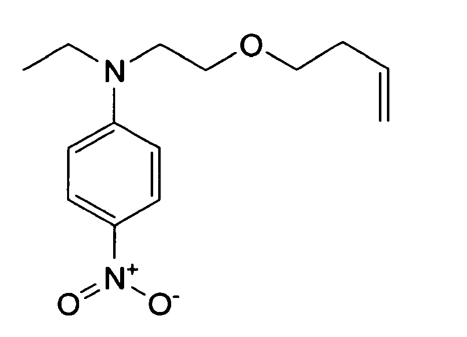 Verbindung 1: N-(2-(but-3-enyloxy)ethyl)-N-ethyl-4-nitrobenzenamin
mit: R¹ = C₂H₄, R² = C₄H₇, R³ = C₂H₅, R⁴ = H und X = O hergestellt. Die Synthese der Verbindung 1 beginnt mit der Umsetzung des kommerziell beziehbaren 1-Fluor-4-nitrobenzols mit dem ebenfalls kommerziell erhältlichen 2-Ethylaminoethanol gemäß dem allgemeinen Reaktionsschema

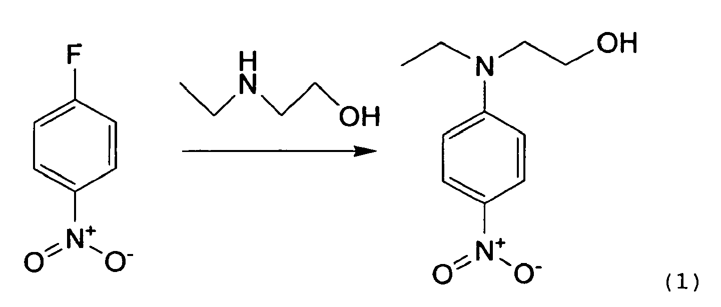

Die erhaltene Zwischenstufe (1) (2-(Ethyl(4-nitrophenyl)amino)ethanol) wird anschließend mit p-Toluolsulfonsäurechlorid gemäß dem allgemeinen Reaktionsschema 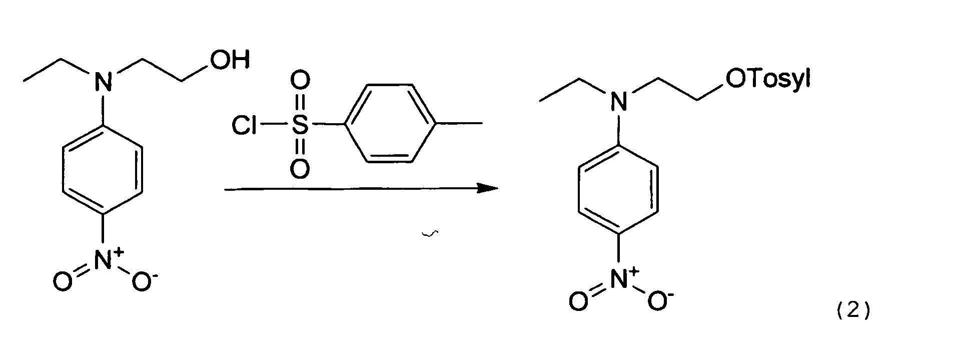 zur Zwischenstufe (2) [2-(Ethyl(4-nitrophenyl)amino)ethyl-4-methylbenzenesulfonat] umgesetzt. Die Verbindung 1 mit einer polymerisierbaren Vinyl-Gruppe wird schließlich durch Substitution der Zwischenstufe (2) mit 1-Hydroxy-3-Buten erhalten:

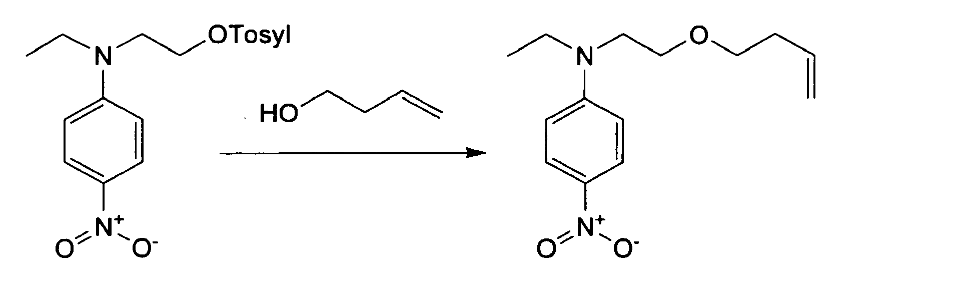

### Beispiel 2

Im zweiten Beispiel für einen gelben Farbstoff der allgemeinen Formel II mit einer Vinylgruppe wird die Verbindung 2 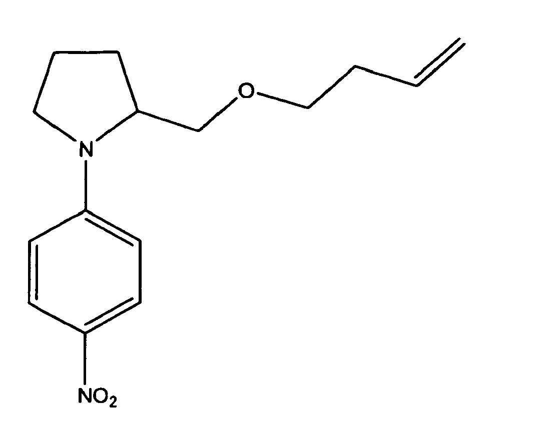 Verbindung 2: 2-((But-3-enyloxy)methyl)-1-(4-nitrophenyl)pyrrolidin mit:
R¹ = CH₂, R² = C₄H₇, R³ und R⁴ = H, n = 0 und X = O
hergestellt. Die Synthese der Verbindung 2 beginnt mit der Umsetzung des kommerziell beziehbaren (1-(4-Nitrophenyl)pyrrolidin-2-yl)methanol mit p-Toluolsulfonsäurechlorid zum Zwischenprodukt (1).

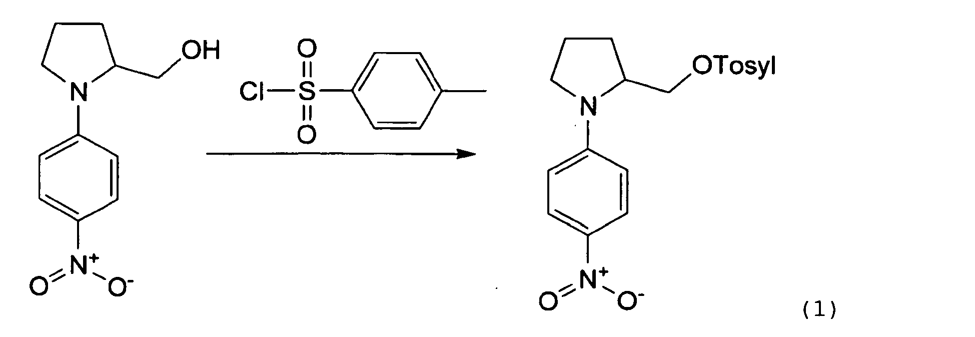

Die Verbindung 2, welche eine polymerisierbare VinylGruppe umfasst, wird durch eine anschließende Substitution des Zwischenprodukts (1) mit 1-Hydroxy-3-Buten erhalten:

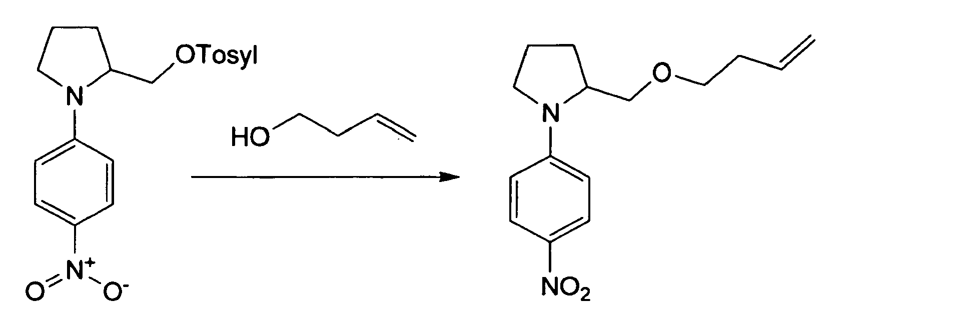

### Detaillierte Synthesebeschreibung:

(S)-[1-(4-Nitrophenyl)pyrrolidin-2-yl]methanol (11,5 g, 51,7 mmol), Tetrabutylammonium-hydrogensulfat (TBAHS, 2,8 g, 8,2 mmol) und pulverisiertes Natriumhydroxyd (14,3 g, 357 mmol) werden in 120 ml Tetrahydrofuran (THF) vereinigt. Anschließend wird zu dem Ansatz bei 5°C-10°C portionsweise p-Touluolsulfonsäurechlorid (11,5 g, 60,3 mmol) innerhalb von 10 Minuten zugegeben. Das Reaktionsgemisch wird während 2 Stunden bei 5°C gerührt.

Zur Aufarbeitung wird das Gemisch mit gesättigter Natriumhydrogenocarbonat-Lösung (75 ml) verdünnt und 10 Minuten bei Raumtemperatur gerührt. Die Phasen werden getrennt. Die wässrige Phase wird mit Dichlormethan (3x15 ml) extrahiert. Die vereinigten organischen Phasen werden mit Na₂SO₄ getrocknet und dann filtriert. Die flüchtigen Bestandteile des Filtrates werden gegebenenfalls im Vakuum vollständig entfernt. Es bleibt das gelbe Zwischenprodukt (1) (Toluol-4-sulfonsäure-(S)-1-(4-Nitrophenyl)pyrrolidin-2-ylmethyl-ester) (21,3 g, ca. 100%) als Feststoff zurück, das ohne weitere Reinigung für die nachfolgende Reaktion verwendet werden kann. Das Zwischenprodukt (1) (21,3 g, ca. 51,7 mmol), Toluol (175 ml), 1-Hydroxy-3-buten (26,2 g, 36,3 mmol), Tetrabutylammoniumhydrogensulfat (5,2 g, 15,3 mmol) und 50-prozentige Natriumhydroxyd-Lösung (70 g, 873 mmol) werden vereinigt. Dann wird das Reaktionsgemisch 90 Minuten bei 65°C bis 70°C gerührt. Zur Aufarbeitung wird das Gemisch mit 65 ml Wasser versetzt. Die Phasen werden getrennt. Die wässrige Phase wird mit Dichlormethan (3x20 ml) extrahiert. Die vereinigten organischen Phasen werden mit Na₂SO₄ getrocknet und dann filtriert. Die flüchtigen Bestandteile des Filtrates können im Vakuum vollständig entfernt werden. Der Rückstand (braunes Öl, 21,6 g) wird säulenchromatographisch gereinigt (Lösungsmittel Cyclohexan/Ethylacetat 11:1). Das erhaltene gelbe Öl (8,58 g) kann vorzugsweise über Nacht kristallisiert werden. Zur Feinreinigung wird das Produkt zweimal aus Methanol umkristallisiert. Die Kristalle werden in Methanol (50 ml) bei Raumtemperatur aufgelöst. Die Lösung wird anschließend langsam auf -55°C bis -60°C abgekühlt und dann 10 Minuten gerührt. Die ausgefallenen Kristalle werden abgesaugt und mit kaltem Hexan (-75°C, 3x10 ml) gewaschen. Diese Prozedur wurde nochmals wiederholt. Die Verbindung 2 ((S)-2-But-3-enyloxymethyl-1-(4-nitrophenyl)pyrrolidin) wird als gelber Feststoff in einer Ausbeute von 57% (8,20 g) mit einem Schmelzpunkt von 35-36°C erhalten (HPLC-Reinheit 99,7%). Es ist jedoch zu betonen, dass die Synthese nicht mit enantiomerenreinen Edukten durchgeführt werden muss und auch racemische Gemische verwendet werden können.

### Beispiel 3

Im dritten Beispiel für einen gelben Farbstoff der allgemeinen Formel I mit einer Methacrylat-Gruppe wird die Verbindung 3 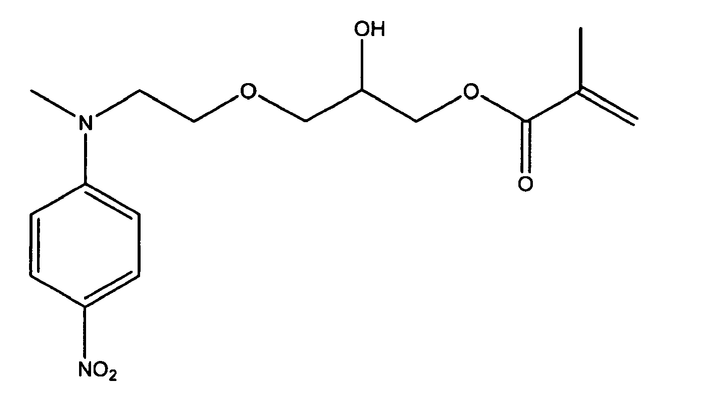 Verbindung 3: 2-Hydroxy-3-(2-(methyl(4-nitrophenyl)-amino)ethoxy)propylmethacrylat mit:
R¹ = C₅H₁₀O₂, R² = C₄H₅O, R³ = CH₃, R⁴ = H und X = O
hergestellt. Die Synthese der Verbindung 3 beginnt wiederum mit der Umsetzung des kommerziell beziehbaren 1-Fluor-4-nitrobenzols mit dem kommerziell erhältlichen 2-Methylaminoethanol zum Zwischenprodukt (1) [2- (Methyl (4-nitrophenyl)amino)ethanol].

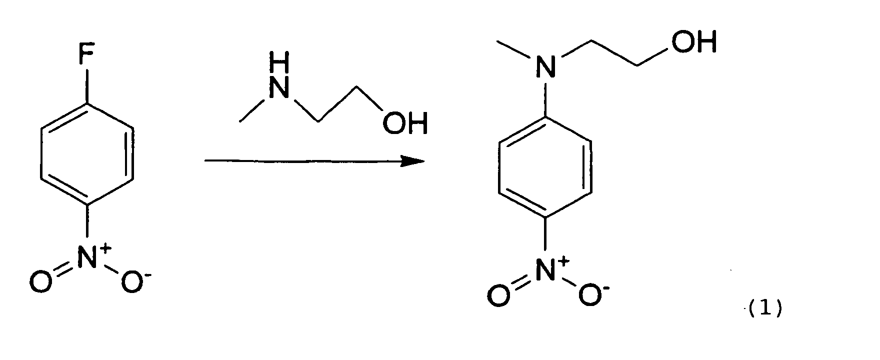

Das erhaltene Zwischenprodukt (1) wird anschließend mit Methacrylsäureglycidylester zur Verbindung 3 umgesetzt:

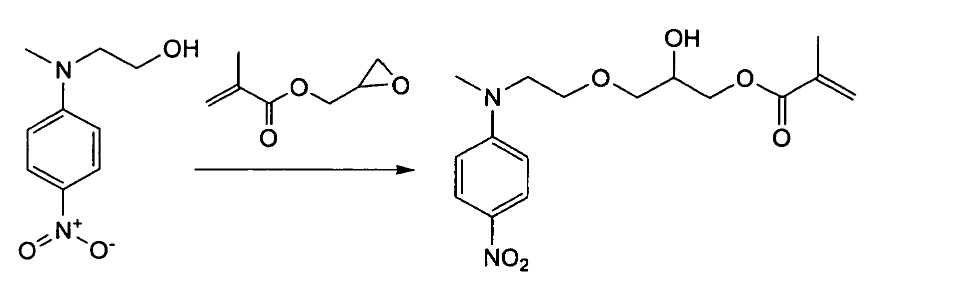

### Beispiel 4

Im vierten Beispiel für einen gelben Farbstoff der allgemeinen Formel II mit einer Methacrylat-Gruppe wird die Verbindung 4: 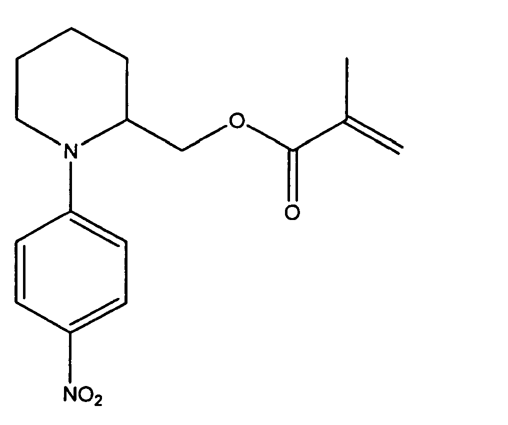 Verbindung 4: (1-(4-Nitrophenyl)piperidin-2-yl)-methylmethacrylat
mit:
R¹ = CH₂, R² = C₄H₅O, R³ und R⁴ = H, n = 1, R⁵ = H und X = O hergestellt.

Die Synthese der Verbindung 3 beginnt mit der Umsetzung des kommerziell beziehbaren 1-Fluor-4-nitrobenzols mit dem ebenfalls kommerziell erhältlichen Piperidin-2-methanol zum Zwischenprodukt (1) ((1-(4-nitrophenyl)piperidin-2-yl)methanol).

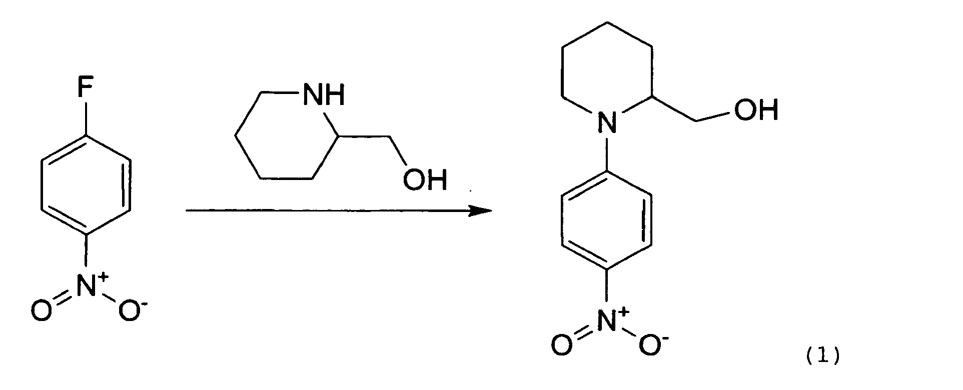

Das erhaltene Zwischenprodukt (1) wird dann mit Methacrylsäurechlorid zur Verbindung 4 verestert.

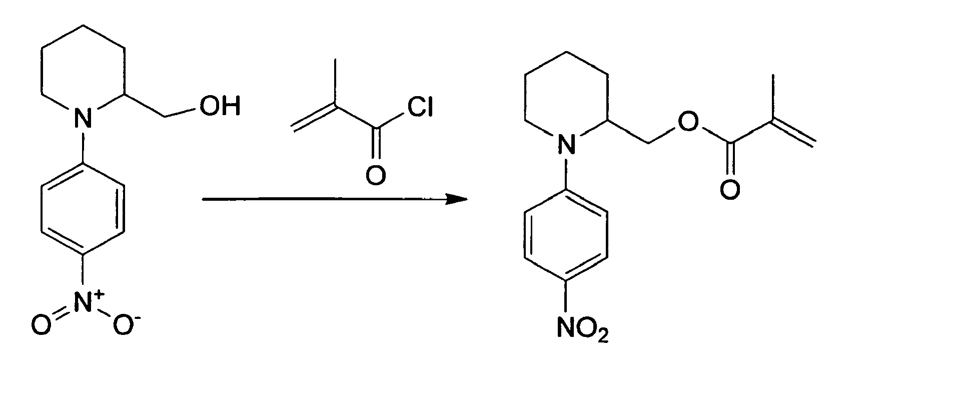

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

## Patentansprüche

1. Ophthalmologische Zusammensetzung mit einem Farbstoff der
allgemeinen Formel I 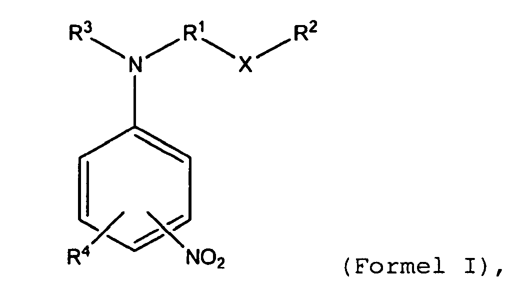 wobei der Farbstoff der allgemeinen Formel I die Struktur
oder die Struktur 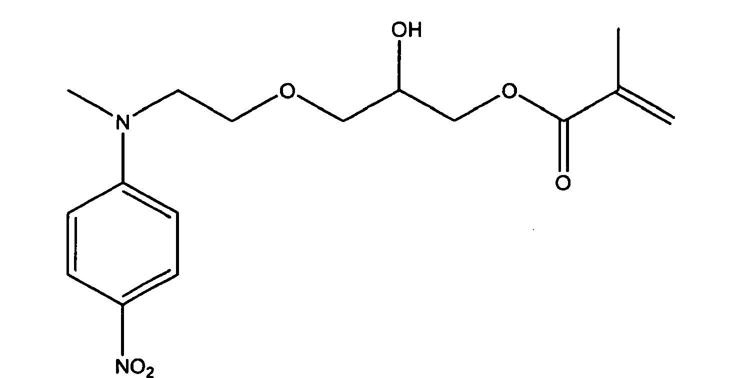 besitzt.

2. Ophthalmologische Zusammensetzung mit einem Farbstoff der allgemeinen Formel II 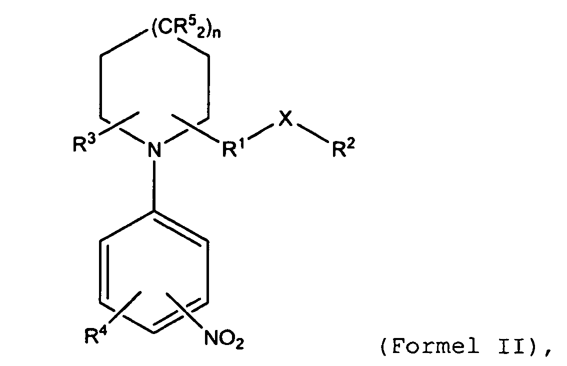 wobei der Farbstoff der allgemeinen Formel II die Struktur 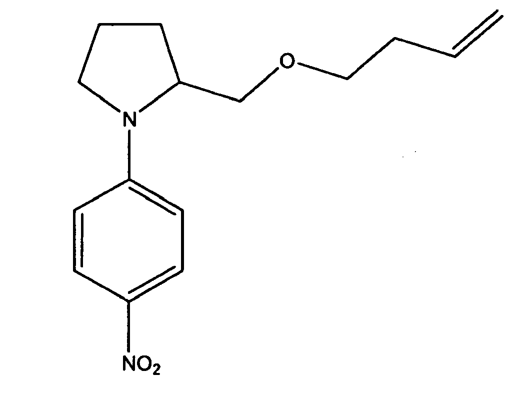 oder die Struktur 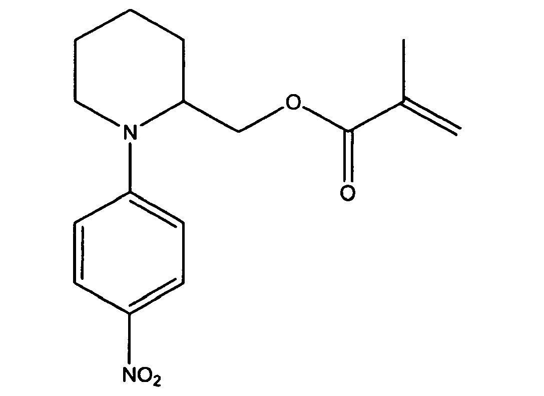 besitzt.

3. Ophthalmologische Zusammensetzung nach einem der Ansprüche 1 oder 2, bei welcher der Farbstoff kovalent in ein Polymer eingebunden ist.

4. Ophthalmologische Zusammensetzung nach einem der Ansprüche 1 bis 3, welche ein Monomer gemäß der nachfolgenden allgemeinen Formel III 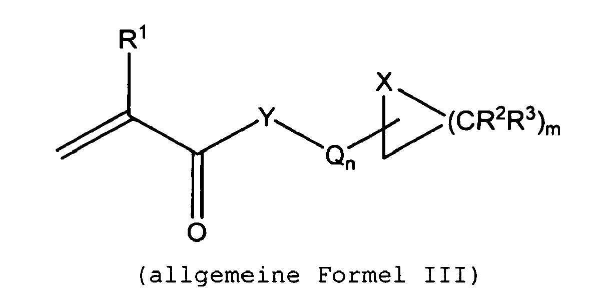 umfasst, wobei
R¹, R² und R³ jeweils unabhängig voneinander Wasserstoff oder Alkyl- bedeuten,
Y: O oder NR⁴ mit R⁴ ausgewählt aus Wasserstoff oder Alkylbedeutet,
X: O, S, SO oder SO₂ bedeutet,
Q: eine Struktureinheit ausgewählt aus CHR⁵ oder (CHR⁵CHR⁶O)ᵢCH₂ bedeutet, wobei R⁵ und R⁶ jeweils unabhängig voneinander Wasserstoff oder Alkyl- bedeuten,
n und i unabhängig voneinander eine ganze Zahl zwischen 1 und 10 und
m eine ganze Zahl zwischen 2 und 6 bedeuten.

5. Ophthalmologische Zusammensetzung nach einem der Ansprüche 1 bis 4, umfassend:
- 50-90%, vorzugsweise 70-85%, HEMA;
- 10-40%, vorzugsweise 15-30%, MMA;
- 0,25-1,5%, vorzugsweise 0,4%-0,6%, Vernetzer;
- 0,01-0,3% Radikalstarter;
- 0,1-3,0%, vorzugsweise 0,70-0,80%, UV-Absorber; und
- 0,02-0,3%, vorzugsweise 0,15-0,17%, Farbstoff.

6. Ophthalmologische Linse, umfassend eine ophthalmologische Zusammensetzung nach einem der Ansprüche 1 bis 5.

7. Ophthalmologische Linse nach Anspruch 6, **dadurch gekennzeichnet, dass** diese als Intraokularlinse oder als Kontaktlinse ausgebildet ist.

## Claims

1. An ophthalmologic composition including a dye of the general formula I 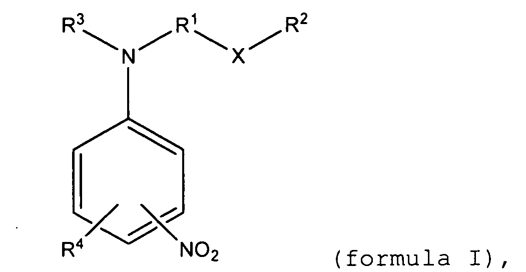 in which the dye of the general formula I has the structure 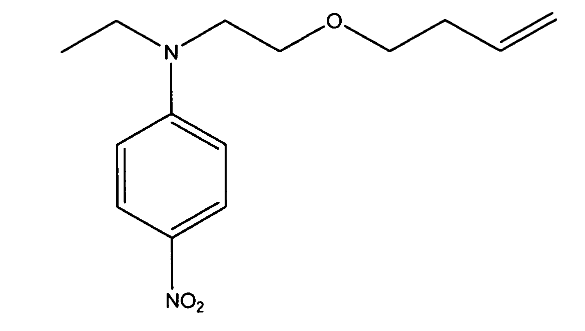 or the structure

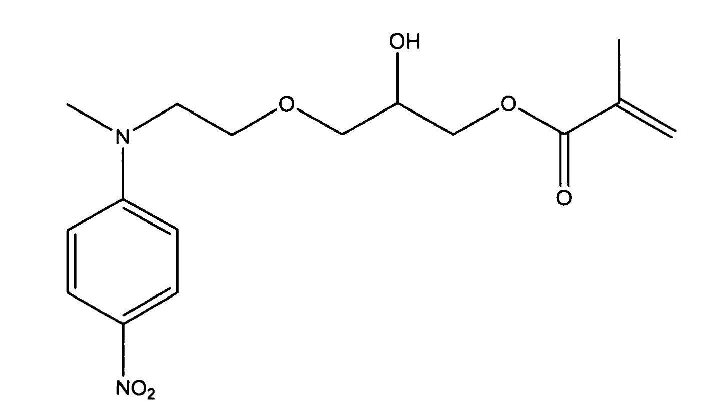

2. An ophthalmologic composition including a dye of the general formula II 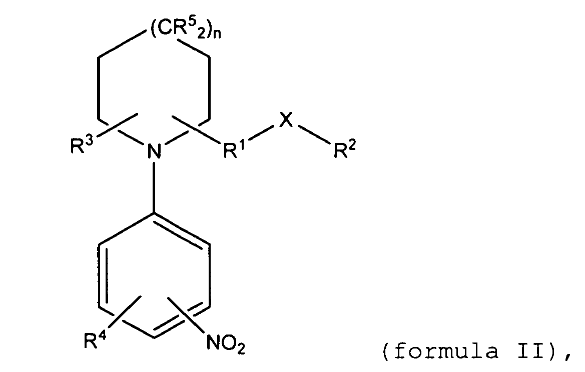 in which the dye of the general formula II has the structure 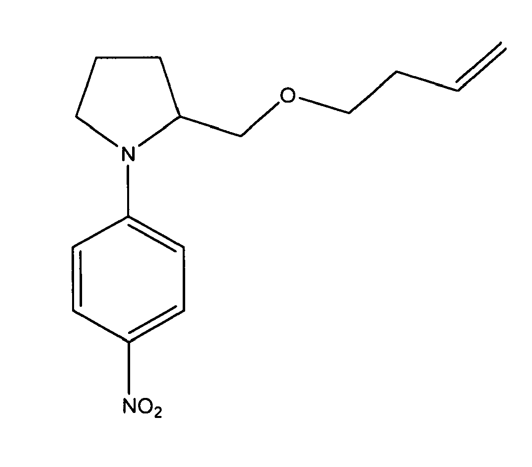 or the structure

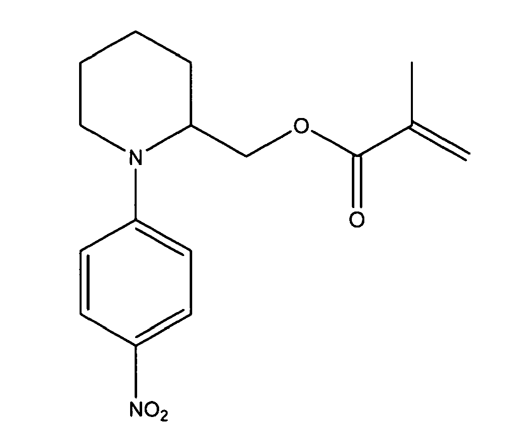

3. The ophthalmologic composition according to any one of claims 1 or 2, in which the dye is covalently incorporated in a polymer.

4. The ophthalmologic composition according to any one of claims 1 to 3, which includes a monomer according to the following general formula III 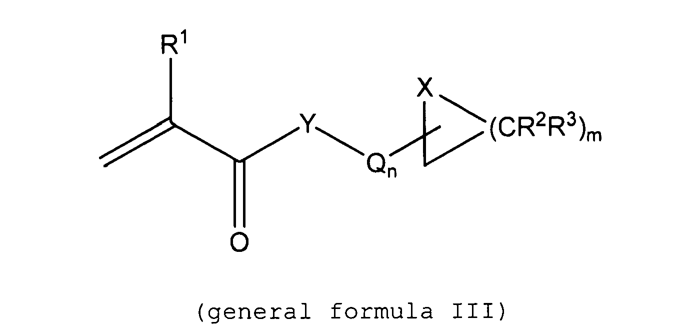 wherein
R¹, R², and R³ each independently of each other denote hydrogen or alkyl,
Y: denotes 0 or NR⁴ with R⁴ selected from hydrogen or alkyl,
X: denotes O, S, SO, or SO₂,
Q: denotes a structural unit selected from CHR⁵ or (CHR⁵CHR⁶O) ᵢCH₂, wherein R⁵ and R⁶ each independently of each other denote hydrogen or alkyl,
n and i independently of each other denote an integer between 1 and 10, and
m denotes an integer between 2 and 6.

5. The ophthalmologic composition according to any one of claims 1 to 4, including:
- 50-90%, preferably 70-85%, HEMA;
- 10-40%, preferably 15-30%, MMA;
- 0.25-1.5%, preferably 0.4%-0.6%, cross-linker;
- 0.01-0.3% radical initiator;
- 0.1-3.0%, preferably 0.70-0.80%, UV absorber; and
- 0.02-0.3%, preferably 0.15-0.17%, dye.

6. An ophthalmologic lens including an ophthalmologic composition according to any one of claims 1 to 5.

7. The ophthalmologic lens according to claim 6, **characterized in that** it is formed as an intraocular lens or as a contact lens.

## Revendications

1. Composition ophtalmologique avec une matière colorante de la formule générale I 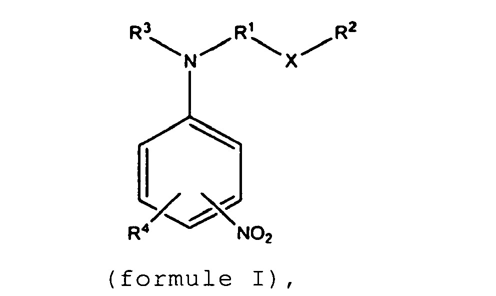 moyennant quoi la matière colorante de la formule générale I possède la structure 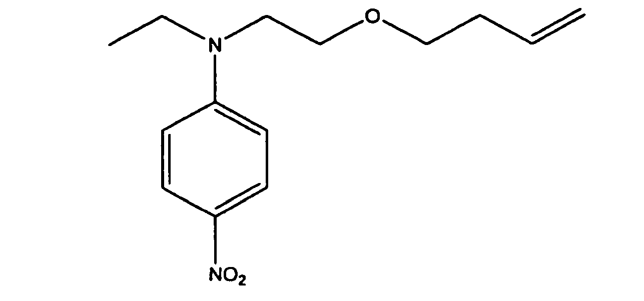 Ou la structure

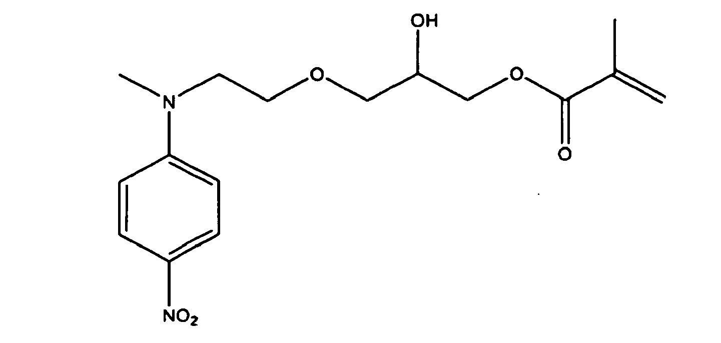

2. Composition ophtalmologique avec une matière colorante de la formule générale II 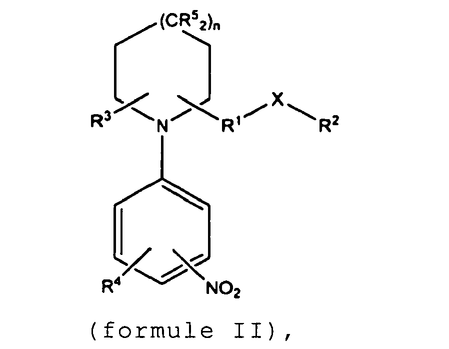 moyennant quoi la matière colorante de la formule générale II possède la structure 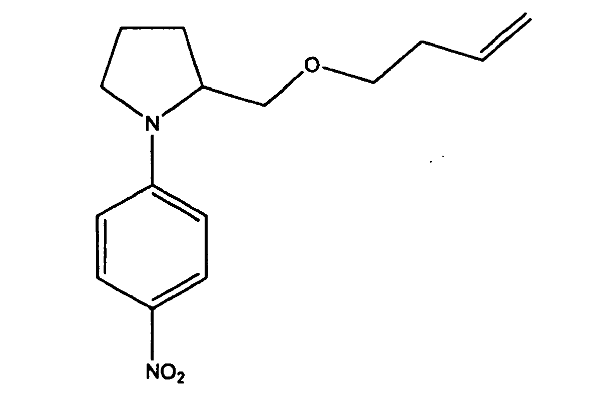 ou la structure

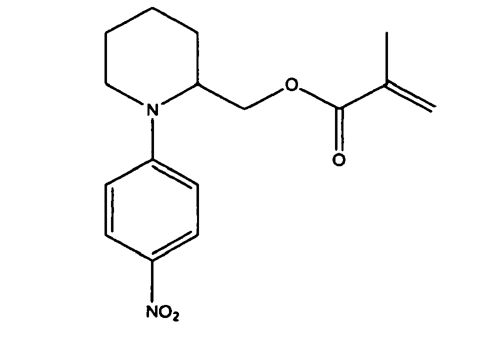

3. Composition ophtalmologique suivant l'une des revendications 1 ou 2, pour laquelle la matière colorante est intégrée de manière covalente dans un polymère.

4. Composition ophtalmologique suivant l'une des revendications 1 à 3, laquelle comprend un monomère, conformément à la formule générale III ci-après 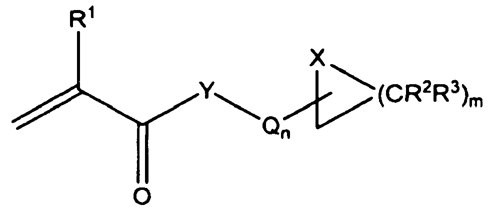 (formule générale III), moyennant quoi
R¹, R² et R³ signifient, respectivement indépendamment l'un de l'autre, de l'hydrogène ou de l'alkyle,
Y : signifie O ou NR⁴ avec R⁴, sélectionné à partir de l'hydrogène ou de l'alkyle-,
X : signifie 0, S, SO ou SO₂,
Q : signifie une unité structurale, sélectionnée à partir de CHR⁵ ou de (CHR⁵CHR⁶O) ᵢCH₂, moyennant quoi R⁵ et R⁶ signifient, respectivement indépendamment l'un de l'autre, de l'hydrogène ou de l'alkyle,
n et i signifient, indépendamment l'un de l'autre, un nombre entier entre 1 et 10 et
m signifie un nombre entier entre 2 et 6.

5. Composition ophtalmologique suivant l'une des revendications 1 à 4, comprenant :
- 50 à 90 %, de préférence 70 - 85 % de HEMA,
- 10 à 40 %, de préférence 15 à 30 % de MMA,
- 0,25 à 1,5 %, de préférence 0,4 % à 0,6 % de réticuleur,
- 0,01 à 0,3 % de démarreur radicalaire,
- 0,1 à 3,0 %, de préférence 0,70 à 0,80 % d'absorbeur d'UV et
- 0,02 à 0,3 %, de préférence 0,15 à 0,17 % de matière colorante.

6. Lentille ophtalmologique, comprenant une composition ophtalmologique suivant l'une des revendications 1 à 5.

7. Lentille ophtalmologique, suivant la revendication 6, **caractérisée en ce que** celle-ci est formée en tant que lentille intraoculaire ou que lentille de contact.
